# EUROPEAN PATENT SPECIFICATION

(11) **EP 1 171 190 B1**
(45) Date of publication and mention of the grant of the patent: **04.05.2011**
(21) Application number: 00925875.7
(22) Date of filing: 22.03.2000
(51) Int. Cl.: A61N 1/375, C04B 37/02

(54) **CERAMIC CASE ASSEMBLY FOR A MICROSTIMULATOR**
KERAMISCHE GEHÄUSEEINHEIT FÜR MIKROSTIMULATOR
ASSEMBLAGE D'ETUI EN CERAMIQUE DESTINE A UN MICROSTIMULATEUR

(30) Priority: 24.03.1999 US 125872 P
(43) Date of publication of application: 16.01.2002
(73) Proprietor: Alfred E. Mann Foundation, Santa Clarita, CA 91380-9005 (US); Schulman, Joseph H., Santa Clarita, CA 91351 (US)
(72) Inventor: SCHULMAN, Joseph, H., Santa Clarita, CA 91351 (US)
(74) Representative: Paget, Hugh Charles Edward
(86) International application number: PCT/US2000/007555
(87) International publication number: WO 2000/056394

(56) References cited:
- EP-A- 0 380 200
- GB-A- 1 024 210
- US-A- 4 785 827
- US-A- 4 991 582
- US-A1- 4 785 827

## Description

### FIELD OF THE INVENTION

The present invention relates to a structure and method of manufacture of a ceramic case assembly for a microstimulator or microsensor for implantation in a living body, and more particularly to a ceramic case that is of a size and shape capable of implantation by expulsion through the lumen of a hypodermic needle.

### BACKGROUND OF THE INVENTION

Simulators that are implanted in living bodies and powered from external sources must be housed in packages constructed of biocompatible materials. These packages must protect the electronic circuitry located within them from body fluids and ions so that the electronic circuitry can survive for extended periods of time without any significant changes in performance.

Today, the most commonly used metals for implantable packages are titanium, stainless steel and cobalt-chromium alloys. These metals are biocompatible and corrosion resistant. Normally, the package construction consists of parts that are welded together to ensure hermeticity. However, where there is a need to inductively couple an alternating electromagnetic field to an internal pickup coil, the metal package becomes a hindrance. Specifically, transmission of power is substantially reduced by eddy currents generated in the metal package due to the alternating electromagnetic field. To solve that problem, receiving coils are often placed outside the metal package, increasing the size and complexity of the implanted device.

Electrical stimulation devices contain electrical components inside the package that are connected to stimulation leads by hermetic feedthroughs, which permit the flow of electrical currents through the package wall while maintaining hermeticity. Disadvantageously, each feedthrough is a possible leak path that can ruin the hermeticity of the package.

Glasses and ceramics represent viable materials for an implantable medical device package because they are transparent to alternating electromagnetic fields. Receiving coils can be placed inside a hermetic zone of a ceramic or glass package, creating an overall smaller and simpler implant device and reducing the possibility of coil failure due to saline leakage. Glasses and ceramics are inert and highly insoluble, which are favorable characteristics for long term implant materials.

Unfortunately, all known biocompatible glasses and ceramics are characterized by high sealing temperatures which high temperatures may damage electronic components commonly included in electronic devices implanted in living bodies. Low-melting temperature glasses may not be used because they all have the property of being corroded by body fluids. Therefore, packages composed entirely of ceramic and/or glass have generally not been considered practical for implant applications. Also, because glasses and ceramics are inelastic, they are subject to fracture not only from mechanical shock but also from differential thermal expansion if even a moderate temperature gradient exists thereacross. Therefore, welding is not a practical method of sealing glass or ceramic materials. Instead, if a glass package is used, virtually the entire package and its contents must be raised to the high melting temperature of the glass, ceramic or metal braze that is used to effect a sealing of the glass or ceramic package. Such sealing methods are unsatisfactory.

One type of hermetically sealed ceramic and metal package is shown in U.S. Patent No. 4,991,582, issued to Byers, et al. A ceramic case and a metal band are hermetically sealed together, each being characterized by similar coefficients of linear thermal expansion. The electronic circuitry is then loaded inside the package, and soldering a metal header plate to the metal band effects final package closure.

A brazeless ceramic-to-metal bond for use in implantable devices is shown in U.S. Patent No. 5,513,793 issued to Malmgren. The '793 patent describes a method and apparatus for forming a hermetically sealed bond between a ceramic case and a metal band. The ceramic case and the metal band are hermetically sealed together at elevated temperature and pressure. The ceramic and metal thus bonded are characterized by similar coefficients of linear thermal expansion.

The structure and method of manufacture of an implantable microstimulator are shown in U.S. Patent Nos. 5,193,539 and 5,193,540, both issued to Schulman et al. These patents describe an implantable microstimulator which is substantially encapsulated within a hermetically sealed housing inert to body fluids. The housing is made of glass capillary tubing with electrodes exposed at each end, the electrodes being sealed to the glass tube with glass beads.

Unfortunately, the microstimulator shown in the '539 and 540 patents is difficult to manufacture; the external electrodes are fragile and require many welds to hermetically seal the package. These welds are done one at a time and are very time consuming. The joint between the glass case, glass beads and metal electrodes is also susceptible to leaking. Since the microstimulator case is made from glass, it is fragile and susceptible to cracking. Disadvantageously, glass is also transparent to light. Some components inside a glass package may be light sensitive and, if so, a light barrier must be provided, such as a film or mask covering the components to prevent undesired light from reaching the components.

Malmgren, Santogrossi, and Schulman show a method and apparatus of a strong metal-to-ceramic bond (WO 0056677). A device according to the preamble of claim 1 is known from US4785827.

In view of the above, it is evident that what is needed is a microstimulator package that is constructed from a material that is transparent to an alternating magnetic field. It must at the same time protect the electronic circuitry hermetically sealed therein. It must minimize the number of joints to be sealed, it must not be prone to cracking or leaking, and it must be cost effective to manufacture.

### SUMMARY OF THE INVENTION

The present invention advantageously addresses the needs above as well as other needs by providing an apparatus and method for manufacturing a ceramic case assembly for a microstimulator as defined in claims 1 and 8 respectively.

The ceramic case assembly of the present invention is inert to body fluids. The majority of the case is made of biocompatible ceramic material that is cylindrical in shape with open ends, i.e., tube-shaped. A closed metal band or ring is attached to one or both ends of ceramic case. The metal band is made from a biocompatible material that has the same coefficient of thermal expansion (CTE) as the ceramic material. The attachment of the metal band to the ceramic case can either be a butt joint or one of the components may have a fixturing ring, step or angle for self-jigging (with the other surface with the appropriate mate able surface). The attachment also provides a hermetic seal. The method of attachment is brazing the metal band to the ceramic case. Such brazing, while performed at a high temperature, is done without any electronic circuitry being present. It results in a hermetic seal. Once the brazing is done, the ceramic case assembly is ready for assembly with other components (i.e., electronic circuitry, end caps) to make a hermetically sealed microstimulator. Welding the metal end cap to the metal ring completes the hermetic seal. Normally the weld is made about 0.100 inches away from the braze, with a heat sink, or heat sinks, in use so as to keep the braze from melting. Once thus assembled, the microstimulator with ceramic case is ready to be implanted in a living body.

### BRIEF DESCRIPTION OF THE DRAWINGS

The above and other aspects, features and advantages of the present invention will be more apparent from the following more particular description thereof, presented in conjunction with the following drawings wherein:
Figure 1 is a cross-sectional exploded view of a ceramic case housing assembly including a ceramic case and a metal band;
Figure 2A is a cross-sectional view of the ceramic case housing assembly showing the ceramic case and the metal band assembled together with the subsequently inserted electronic circuit assembly;
Figure 2B is a cross-sectional view of the ceramic case housing assembly showing the ceramic case and the metal band assembled together with the subsequently inserted electronic circuit assembly together with the assembly second metal cap and showing the use of heat sinks;
Figure 3A is a partial cross-sectional view of another embodiment of Figures 2A and 2B showing an optional technique for attaching the metal band to the ceramic case using a step joint for self jigging;
Figure 3B is a partial cross-sectional view of another embodiment of Figures 2A and 2B showing a different optional technique for attaching the metal band to the ceramic case using two mate-able surfaces each at an appropriate to form a self jigging joint;
Figure 4 is a cross-sectional view of another embodiment of Figures 2A and 2B showing a metal band attached at each end of the ceramic case; and
Figure 5 is a cross-sectional exploded view of a ceramic case housing assembly including a ceramic case and a metal band, two metal end caps, and two high temperature braze preforms in the shape of rings.

Below is a list of reference numbers associated with the figures.

| No. | Component |
|---|---|
| 10 | Housing Assembly |
| 12 | Ceramic Case |
| 14 | Metal Band |
| 18 | Bonding Site |
| 22 | Wall of the Ceramic Case |
| 24 | Flat Annular Surface (Ceramic Case) |
| 26 | Flat Annular Surface (Ceramic Case) |
| 28 | Wall of Metal Ring |
| 30 | Flat Annular Surface (Metal Band) |
| 32 | Flat Annular Surface (Metal Band) |
| 101 | Electronic Circuit Assembly |
| 102 | High Temperature Braze |
| 103 | Second Metal Cap |
| 104 | First Metal Cap |
| 201 | High Temperature Braze Preform |
| 202 | High Temperature Braze Preform |

### DETAILED DESCRIPTION OF THE PREFERRED EMBODIMENTS

The following description of the presently contemplated best mode of practicing the invention is not to be taken in a limiting sense, but is made merely for the purpose of describing the general principles of the invention. The scope of the invention should be determined with reference to the claims.

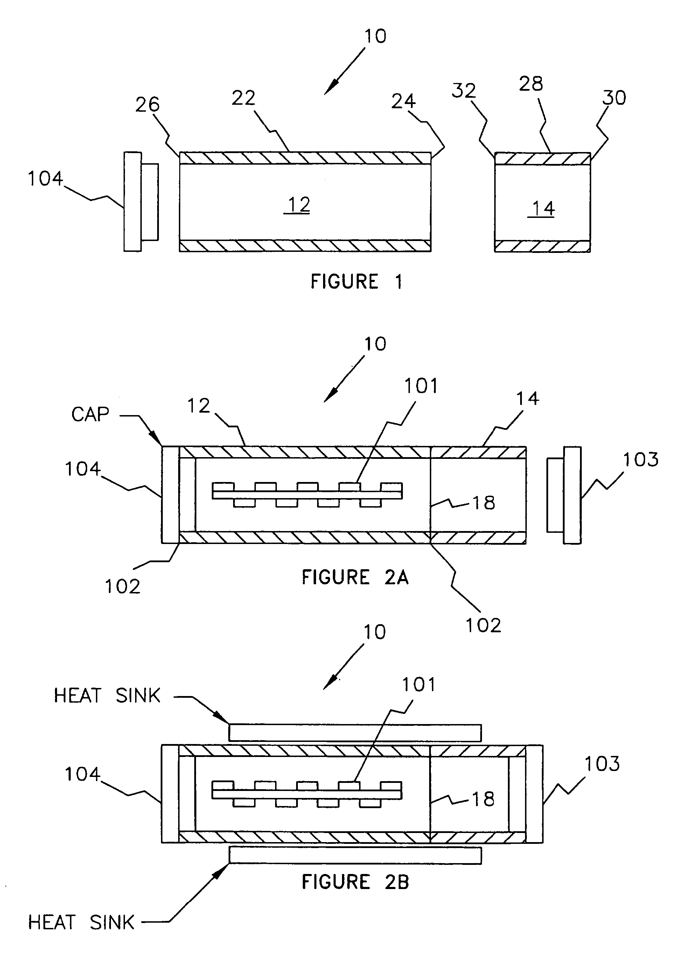

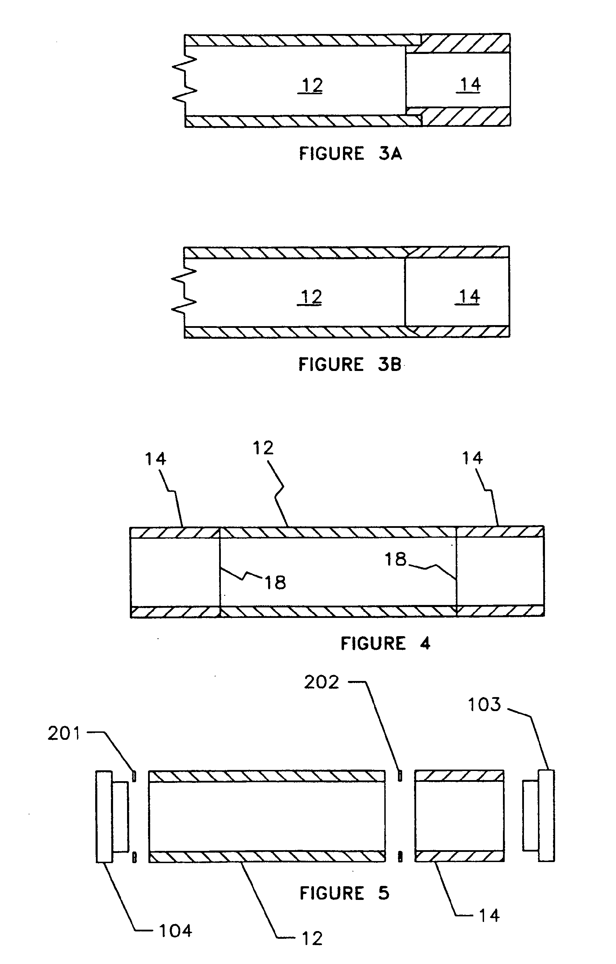

Figures 1, 2A and 2B show the construction of the ceramic case housing assembly 10. Referring first to Figure 1, a cross-sectional exploded view is shown of the components used in the housing assembly 10. These components included a ceramic case 12 and a metal band 14. Figures 2A and 2B are cross-sectional views showing the assembled housing assembly 10. In Figures 2A and 2B, the ceramic case 12 and the metal band 14 are joined together with a hermetically sealed bond, such as a metal or metal alloy braze (e.g., nickel and titanium braze, 30/70), shown as bonding site 18. Since braze bonding is done at temperatures (over 1000 °C) and' pressures (over 100 PSI) that can damage electronic circuitry, this circuitry is only inserted into the housing assembly 10 after the attachment of the metal band 14 to the ceramic case 12 is completed. The ceramic case 12 and the metal band 14 have similar diameters with a butt attachment. Figure 3A shows another attachment method of the band 14 to the case 12 that could be used, such as a step for self-jigging. Figure 3B shows another attachment method of the band 14 to the case 12 that could be used, such as a bezel for self-jigging. The approaches of both Figures 3A and 3B make it possible to increase the braze distance to ensure hermeticity while decreasing the wall thickness of the metal or ceramic part.

The ceramic case 12 is made from the biocompatible ceramic material, zirconium oxide. The case 12 is cylindrical in shape (i.e., a hollow tube) with openings at both ends. The diameter of the ceramic case 12 is typically about 2 mm and the length is approximately 10 mm. The side wall 22 of the ceramic case 12 terminates around the open ends forming flat annular surfaces 24 and 26 at each end. Since the ceramic is not transparent, it tends to protect internal components that are sensitive to light.

The metal band 14 is preferable made from a biocompatible metal and is cylindrical in shape with openings at both ends (i.e., a ring). The diameter of the metal band 14 is 2 mm in diameter and is at least 1 mm in length, and preferably approximately 2.5 mm in length. The side wall 28 of the metal band 14 terminates at each of the open ends, forming flat annular surfaces 30 and 32, to which the flat annular surface 24 of the ceramic case 12 is ultimately bonded at bonding site 18 (as shown in Figures 2A and 2B). The band 14 is made from biocompatible metal, e.g. titanium, stainless steel, tantalum, niobium and niobium-titanium alloys such as 45% niobium-55% titanium and cobalt-chromium alloys. A preferred embodiment of band 14 uses any metal or alloy that readily forms an instant oxide when heated, i.e., that readily oxidizes when heated in an oxygen-containing atmosphere. Note that the ceramic, the metal and the braze are chosen to have similar coefficients of thermal expansion (CTE) of between 8 and 9 mm³/°C. This minimizes the risk of cracking when the ceramic case 12, metal band 14 and braze are bonded together at high temperatures and then cooled.

Figure 2A shows the assembled ceramic case housing assembly 10. Many well-known processes may be used to hermetically bond the metal band 14 to the end of the ceramic case 12 utilizing biocompatible sealing materials. Some of these methods are described in U.S. Patent Nos. 4,991,582 and 5,513,793.

In a preferred method which is not part of the invention, the housing assembly 10 is manufactured by brazing the metal band 14 to the aluminum oxide ceramic case 12 with a nickel-titanium (NiTi) braze with 30%Ni-70%Ti. A best mode metal band, or ferrule, is one made of niobium. The brazing of a zirconium oxide ceramic case, including 3% yttrium as a best mode formulation, utilizes a braze of 50% Ni and 50% Ti, with the metal band, or ferrule, in a best mode, made from a 45% niobium-55% titanium alloy.

The brazing operation may be done in a production line operation to manufacture more than one housing assembly 10 at a time. Since brazing 102 temperatures may reach over 1000 °C, any electronic circuitry assembly (see 101 in Figures 2A, 2B) for a microstimulator is inserted into the housing assembly 10 only after the bonding at such high temperature is completed. Figure 2B shows the use of heat sinks when attaching the second cap 103. The first cap 104 is first attached to the case with a high temperature braze 102 before the insertion of the electronic circuit assembly 101.

Referring next to Figure 4, there is shown another embodiment of the present invention wherein a metal band 14 is attached at each end of the ceramic case. The metal bands are brazed to the ceramic case at bonding sites 18. The attachment may either be a butt or step joint as previously described.

Figure 5 shows the use of braze preforms in the shape of rings (201, 202) sized to fit. In examples not part of the invention, these preforms may be of the braze 30% Ni -70% Ti, or other suitable brazes (in terms of coefficient of expansion and ability to form a hermetic seal). In Figure 5, 104 is a metal cap, 12 is a ceramic cylinder, and 14 is a metal cylinder.

While the invention herein disclosed has been described by means of specific embodiments and applications thereof, numerous modifications and variations could be made thereto by those skilled in the art without departing from the scope of the invention set forth in the claims.

## Claims

1. A ceramic case assembly for a microstimulator comprising:
a cylindrical ceramic case (12, 22) having at least one open end for receiving an electronic circuit assembly of a microstimulator;
a closed annular metal band (14, 28) hermetically bonded to the open end of the ceramic case, the band being formed from a metal having a coefficient of thermal expansion substantially the same as that of the ceramic case, wherein the annular metal band is hermetically bonded to one open end of the ceramic case by a metal braze; **characterized in that**
the metal braze comprises 50% nickel and 50% titanium and the ceramic case is comprised of zirconium oxide.

2. The ceramic case assembly according to claim 1, wherein the cylindrical ceramic case (12) has plural open ends for receiving the electronic circuit assembly of a microstimulator, and a pair of closed annular metal bands (14) which are hermetically bonded to the respective open ends of the ceramic case by said metal braze.

3. The ceramic case assembly of claim 1 or 2, wherein the or each closed metal band (28) is comprised of niobium.

4. The ceramic case assembly of claim 1 or 2, wherein the or each closed metal band is an alloy of 45% niobium - 55% titanium.

5. The ceramic case assembly of any one of claims 1 to 4, wherein the ceramic case comprised of zirconium oxide additionally comprises 3% yttrium.

6. The ceramic case assembly of any one of claims 1 to 5, wherein open end or ends of the ceramic case and the respective metal band or bands butt against each other during bonding forming a butt joint.

7. The ceramic case assembly of any one of claims 1 to 6, wherein the open end or ends of the ceramic case and the respective metal band or bands are shaped according to a form chosen from stepped, or angled, with the appropriate opposite mate-able surface to define a self-jigging junction between the ceramic case and the metal band during bonding, forming a step joint or angle joint.

8. A method of forming a ceramic case assembly for a microstimulator comprising the steps of:
positioning a cylindrical ceramic case against one or more metal bands, the metal band or bands consisting of a second material; **characterized by** the ceramic case consisting of a first material comprised of zirconium oxide with 3% yttrium and by and by
sealing the ceramic case and the or each of the metal bands to the ceramic case hermetically, using a metal braze of 50% nickel and 50% titanium; and
maintaining a tube-like structure for the assembly,
whereby the braze has a coefficient of thermal expansion substantially the same as that of the ceramic case and the closed annular metal band.

## Patentansprüche

1. Keramikgehäuseanordnung für einen Mikrostimulator, die Folgendes umfasst:
ein zylinderförmiges Keramikgehäuse (12, 22) mit zumindest einem offenen Ende zur Aufnahme einer elektronischen Schaltungsanordnung eines Mikrostimulators;
ein geschlossenes ringförmiges Metallband (14, 28), das hermetisch mit dem offenen Ende des Keramikgehäuses durch Haftschluss verbunden ist, wobei das Band aus einem Metall mit einem Wärmeausdehnungskoeffizienten besteht, der im Wesentlichen mit jenem des Keramikgehäuses übereinstimmt, wobei das ringförmige Metallband mittels Metalllötung hermetisch an das offene Ende des Keramikgehäuses angehaftet wird, **dadurch gekennzeichnet, dass**
die Metalllötung 50 % Nickel und 50 % Titan umfasst und das Keramikgehäuse aus Zirkoniumoxid besteht.

2. Keramikgehäuseanordnung nach Anspruch 1, worin das zylinderförmige Keramikgehäuse (12) eine Vielzahl offener Enden, um die elektronische Schaltungsanordnung eines Mikrostimulators aufzunehmen, sowie ein Paar geschlossener ringförmiger Metallbänder (14) aufweist, die mittels Metalllötung an die jeweiligen offenen Enden des Keramikgehäuses hermetisch angehaftet sind.

3. Keramikgehäuseanordnung nach Anspruch 1 oder 2, worin das oder jedes geschlossene Metallband (28) aus Niobium besteht.

4. Keramikgehäuseanordnung nach Anspruch 1 oder 2, worin das oder jedes geschlossene Metallband eine Legierung aus 45 % Niobium und 55 % Titan ist.

5. Keramikgehäuseanordnung nach einem der Ansprüche 1 bis 4, worin das aus Zirkoniumoxid bestehende Keramikgehäuse zusätzlich 3 % Yttrium umfasst.

6. Keramikgehäuseanordnung nach einem der Ansprüche 1 bis 5, worin das offene Ende oder die offenen Enden des Keramikgehäuses und das entsprechende Metallband oder die entsprechenden Metallbänder während der Verbindung aneinander stoßen, um eine Stumpfverbindung zu bilden.

7. Keramikgehäuseanordnung nach einem der Ansprüche 1 bis 6, worin das offene Ende oder die offenen Enden des Keramikgehäuses und das entsprechende Metallband oder die entsprechenden Metallbänder gemäß einer aus abgestuft und winkelig ausgewählten Form mit der geeigneten entgegengesetzten verbindbaren Oberfläche geformt sind, um eine sich selbst erzeugende Verbindung zwischen dem Keramikgehäuse und dem Metallband während des Verbindens zu definieren, wodurch eine überlappende Verbindung oder eine winkelige Verbindung ausgebildet wird.

8. Verfahren zur Ausbildung einer Keramikgehäuseanordnung für einen Mikrostimulator, das folgende Schritte umfasst:
das Anordnen eines zylinderförmigen Keramikgehäuses in Bezug auf ein oder mehrere Metallbänder, wobei das Metallband oder die Metallbänder aus einem zweiten Material bestehen, **dadurch gekennzeichnet, dass** sich das Keramikgehäuse aus einem ersten Material aus Zirkoniumoxid und 3 % Yttrium zusammensetzt und das Keramikgehäuse und das Metallband oder jedes der Metallbänder unter Verwendung einer Metalllötung aus 50 % Nickel und 50 % Titan hermetisch abgedichtet werden, sowie **dadurch**, dass eine röhrenähnliche Struktur der Anordnung aufrechterhalten wird,
wobei die Metalllötung einen Wärmeausdehnungskoeffizienten aufweist, der im Wesentlichen mit jenem des Keramikgehäuses und des geschlossenen ringförmigen Metallbands übereinstimmt.

## Revendications

1. Assemblage de boîtier en céramique pour un microstimulateur comprenant :
un boîtier en céramique (12, 22) cylindrique comportant au moins une extrémité ouverte pour recevoir un assemblage de circuits électroniques d'un microstimulateur ;
une bande métallique (14, 28) annulaire fermée liée hermétiquement à l'extrémité ouverte du boîtier en céramique, la bande étant formée à partir d'un métal ayant un coefficient de dilatation thermique sensiblement identique à celui du boîtier en céramique, dans lequel la bande métallique annulaire est liée hermétiquement à une extrémité ouverte du boîtier en céramique par une brasure de métal ; **caractérisé en ce que**
la brasure de métal comprend 50 % de nickel et 50 % de titane et le boîtier en céramique est composé d'oxyde de zirconium.

2. Assemblage de boîtier en céramique selon la revendication 1, dans lequel le boîtier en céramique (12) cylindrique comporte plusieurs extrémités ouvertes pour recevoir l'assemblage de circuits électroniques d'un microstimulateur, et une paire de bandes métalliques (14) annulaires fermées qui sont liées hermétiquement aux extrémités ouvertes respectives du boîtier en céramique par ladite brasure de métal.

3. Assemblage de boîtier en céramique selon la revendication 1 ou 2, dans lequel la ou chaque bande métallique (28) fermée est composée de niobium.

4. Assemblage de boîtier en céramique selon la revendication 1 ou 2, dans lequel la ou chaque bande métallique fermée est en un alliage de 45 % de niobium et de 55 % de titane.

5. Assemblage de boîtier en céramique selon l'une quelconque des revendications 1 à 4, dans lequel le boîtier en céramique composé d'oxyde de zirconium comprend en plus 3 % d'yttrium.

6. Assemblage de boîtier en céramique selon l'une quelconque des revendications 1 à 5, dans lequel l'extrémité ou les extrémités ouvertes du boîtier en céramique et la bande ou les bandes métalliques respectives sont en butée les unes contre les autres pendant la liaison, formant un joint bout à bout.

7. Assemblage de boîtier en céramique selon l'une quelconque des revendications 1 à 6, dans lequel l'extrémité ou les extrémités ouvertes du boîtier en céramique et la bande ou les bandes métalliques respectives sont mises en forme conformément à une forme choisie parmi une forme étagée, ou une forme angulaire, avec la surface pouvant être accouplée opposée appropriée pour définir une jonction d'auto-assemblage entre le boîtier en céramique et la bande métallique pendant la liaison, formant un joint décroché ou un joint à angle.

8. Procédé de formation d'un assemblage de boîtier en céramique pour un microstimulateur comprenant les étapes consistant à _{:}
positionner un boîtier en céramique cylindrique contre une ou plusieurs bandes métalliques, la ou les bandes métalliques consistant en un deuxième matériau ; **caractérisé en ce que** le boîtier en céramique consiste en un premier matériau composé d'oxyde de zirconium avec 3 % d'yttrium, et
sceller le boîtier en céramique et la ou chacune des bandes métalliques sur le boîtier en céramique hermétiquement, en utilisant une brasure de métal de 50 % de nickel et 50 % de titane ; et
maintenir une structure semblable à un tube pour l'assemblage,
moyennant quoi la brasure a un coefficient de dilatation thermique sensiblement identique à celui du boîtier en céramique et de la bande métallique annulaire fermée.
